# EUROPEAN PATENT APPLICATION

(11) **EP 2 218 407 A1**
(43) Date of publication of application: **18.08.2010**
(21) Application number: 10250270.5
(22) Date of filing: 17.02.2010
(51) Int. Cl.: A61B 17/06, A61B 17/34

(54) **Access port with suture management system including flapper with inserts**

(30) Priority: 17.02.2009 US 153086 P; 14.12.2009 US 636820
(71) Applicant: Tyco Healthcare Group LP, North Haven, CT 06473 (US)
(72) Inventor: Bettuchi, Michael, Middletown, CT 06457 (US)
(74) Representative: Soames, Candida Jane

(57) **Abstract**

An access port provides passage into a subject's body. This access port generally includes a housing and a suture management system. The housing defines a longitudinal axis and has a longitudinal passage. The longitudinal passage is adapted to allow passage of a plurality of sutures through the housing. The suture management system, which is operatively coupled to the housing, includes a flapper having a plurality of spaced apart recesses. Each recess includes an insert disposed in mechanical cooperation therewith, and each insert has a slit configured to receive and hold at least one suture of the plurality of sutures.

## Description

### CROSS REFERENCE TO RELATED APPLICATION

The present application claims the benefit of and priority to U.S. Provisional Application Serial No. 61/153,086 filed on February 17, 2009, the entire contents of which are incorporated herein by reference.

### BACKGROUND

### Technical Field

The present disclosure relates to access ports for providing access into a patient's body. In particular, the present disclosure relates to access ports with a suture managing system configured to hold and segregate sutures.

### DESCRIPTION OF RELATED ART

In laparoscopic procedures, clinicians perform surgery in the interior of the abdomen through a small incision, and in endoscopic procedures, clinicians conduct surgery in any hollow viscus of the body through a narrow tube or cannula inserted through a small entrance incision in the skin. Laparoscopic and endoscopic procedures generally require that any instrumentation inserted into the body be sealed to ensure that gases do not enter or exit the body through the incision. For example, in certain kinds of surgical procedures, the clinician insufflates a surgical region with gases, and a sealed surgical instrument prevents theses gases from escaping the subject's body. In other surgical procedures, such as arthroscopic procedures, the clinician irrigates the target surgical site with saline. Moreover, laparoscopic and endoscopic procedures often require the surgeon to operate on organs, tissue, and vessels far removed from the incision, thereby requiring that any instruments used in such procedures be relatively long and narrow.

In many laparoscopic and endoscopic procedures, clinicians employ a trocar assembly composed of a cannula assembly and an obturator assembly to facilitate the introduction of a tube into certain anatomical cavities such as the abdominal cavity. Since the cannula assembly provides a direct passage for surgical instrumentation from outside the patient's body to the internal organs and tissue, the cannula assembly usually maintains a relatively fluid-tight interface between the abdominal cavity and the outside atmosphere. The cannula assembly generally includes a cannula attached to a cannula housing containing a seal assembly adapted to maintain a seal across the opening of the cannula housing.

Given that surgical procedures in the abdominal cavity require insufflating gases to raise the cavity wall away from vital organs, the procedure is usually initiated by use of a Verres needle through which a gas such as CO₂ is introduced into the body cavity, thus creating a pneumoperitoneum. The gas provides a positive pressure that raises the inner body wall away from internal organs, thereby providing the surgeon with an operating space. By creating an operating space, the clinician avoids unnecessarily contacting the organs with the instruments inserted through the cannula assembly. An obturator of the obturator assembly is inserted into the cannula assembly and used to puncture the abdominal wall. Following removal of the obturator assembly from the cannula assembly, laparoscopic or endoscopic surgical instruments may be inserted through the cannula assembly to perform surgery within the abdominal cavity.

Without the obturator assembly to block the flow of insufflation gas out from the cavity, other structure must be provided to maintain a relatively fluid-tight interface between the abdominal cavity and the outside atmosphere. Insufflatory surgical procedures generally entail two sealing measures. First, the cannula assembly should provide a substantially fluid-tight seal in the absence of a surgical instrument in the cannula. Second, the cannula assembly should provide a substantially fluid-tight seal when the surgical instrument is being introduced into or is already present in the cannula. Additionally, as endoscopic and laparoscopic surgical procedures and techniques have advanced, it has become desirable to accommodate surgical instrumentation of varying outside diameters through a single cannula assembly in a given surgical procedure, thereby minimizing the number of cannulae required and facilitating efficiency in the surgical procedure. It is further desirable to maintain a seal about the instrument for manipulation of the instrument within the cannula assembly.

In certain minimally invasive procedures, such as arthroscopic procedures, clinicians secure soft tissue to a selected bone surface either directly or indirectly via an implant typically called an anchor. During arthroscopic operations, the clinician irrigates the target joint with any suitable irrigation fluid, such as water or saline, to distend the joint, thereby creating space for the surgical operation. These anchors are generally tied to sutures, and the sutures are then tied to the soft tissue to hold it in place. Anchors may be used to attach soft tissue such as ligaments, tendons, muscles, etc. to a bone surface from which the soft tissue has become detached and may also be employed to secure soft tissue to supplementary attachment sites for reinforcement. During these kinds of procedures, clinicians often introduce multiple sutures through the cannula assembly of the trocar assembly. As a result, these sutures may tangle during the surgical procedure. Also, the clinicians may become confused about which suture end corresponds to which suture. To help prevent suture from tangling, certain trocar assemblies feature suture management systems or suture retaining members. Suture management systems usually separate and hold sutures. In doing so, suture management systems also help prevent clinicians from confusing the suture ends of the sutures and provide tension on the suture ends so that the sutures can assist in holding in place certain anatomy within the surgical site. Although several suture management systems have been developed throughout the years, improvements are still possible.

### SUMMARY

The present disclosure relates to an access port for providing passage into a subject's body. This access port generally includes a housing and a suture management system. The housing defines a longitudinal axis and has a longitudinal passage. The longitudinal passage is adapted to allow passage of a plurality of sutures through the housing. The suture management system, which is operatively coupled to the housing, includes a flapper having a plurality of spaced apart recesses. Each recess includes an insert disposed in mechanical cooperation therewith, and each insert has a slit configured to receive and hold at least one suture of the plurality of sutures.

Besides the access port, the present disclosure relates to a method for managing sutures. This methods includes the following steps: (a) providing an access port including a flapper positioned at a proximal end thereof, the flapper having a plurality of spaced apart recesses, each recess including an insert disposed in mechanical cooperation therewith, each inserts having a slit configured to receive and hold at least one suture of a plurality of sutures; (b) positioning at least a portion of the access port inside a subject's body; (c) inserting the plurality of sutures inside the subject's body through the access port; and (d) placing at least a portion of each suture into each slit to hold and separate the sutures from one another.

### BRIEF DESCRIPTION OF THE DRAWING

Figure 1 is a perspective view of an access port including a suture management system with a suture retainer member;

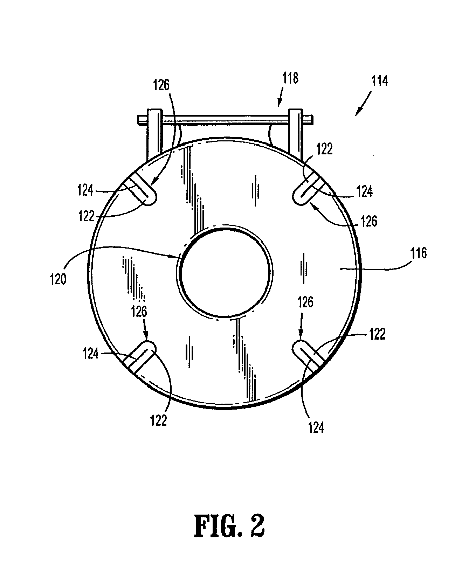

Figure 2 is a top plan view of the retainer member of the access port shown in Figure 1;

Figure 3 is a perspective view of the access port of Figure 1 illustrating a surgical instrument introduced within the retainer member; and

Figure 4 is a side view of the access port of Figure 1 with a retainer member in an open position.

### DETAILED DESCRIPTION OF THE EMBODIMENTS

In the description that follows, the term "proximal" refers to the portion of the access port that is closest to the clinician, while the term "distal" refers to the portion of the access port that is farthest from the clinician. As used herein, the term "subject" refers to a human patient or other animal. The term "clinician" refers to a physician, nurse or other care provider and may include support personnel.

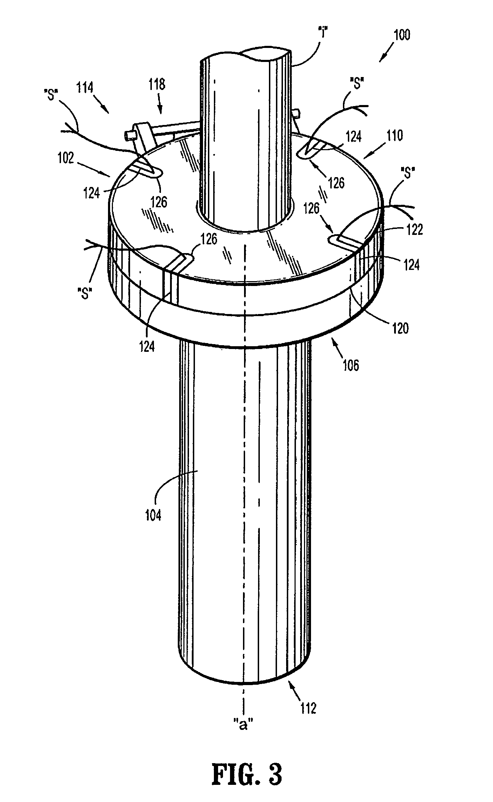

The presently disclosed access port facilitates the introduction of surgical instruments and sutures into a subject's body cavity. Specifically, a clinician may utilize the access port of the present disclosure to provide passage between a subject's body cavity and the outside atmosphere. The access port is configured to receive clip appliers, graspers, dissectors, retractors, staplers, laser probes, photographic devices, endoscopes and laparoscope, tubes, and the like. Such instruments "i" are collectively referred to herein as "instruments" or "instrumentation" (See FIG. 3).

Aside from the instruments, the access port allows passage of one or more sutures. When several sutures are inserted into the subject's body, the sutures may tangle or their arrangement may become confused. Suture tangle and/or confusion may, at the very least, inconvenience the clinician performing the surgical procedure. To minimize the probability of sutures tangling and/or becoming confused with one another, the presently disclosed access port includes a suture management system. This suture management system retains sutures in place and maintains the sutures separated from one another.

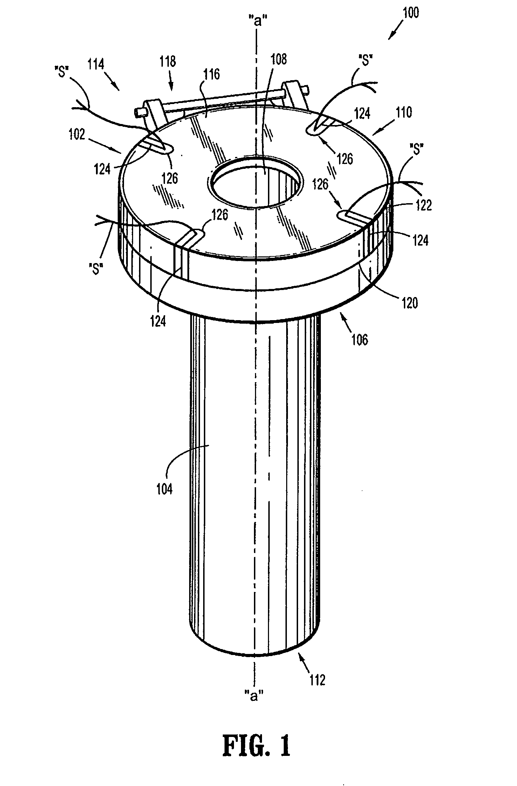

Referring now to the drawings, Figure 1 shows an access port 100 including a housing 102 defining a longitudinal axis "a" extending therethrough and a cannula sleeve 104. Cannula sleeve 104 is attached to or is configured to mount to a distal end 106 of housing 102. Any suitable fastening device, apparatus, or mechanism may be used to secure cannula sleeve 104 to housing 102. For instance, threaded arrangements, bayonet couplings, snap-fit arrangements, adhesives or any other suitable mechanism may connect distal end 106 of housing 102 to cannula sleeve 104. Cannula sleeve 104 may be made of stainless steel, polymeric material, or any other suitable material. These materials may be opaque, translucent or transparent. The diameter of cannula sleeve 104 may vary depending on the procedure in which the cannula sleeve 104 is being utilized, e.g., in an endoscopic procedure, laparoscopic procedure or arthroscopic procedure.

Housing 102 and cannula sleeve 104 together form a longitudinal passage 108 dimensioned to allow passage of surgical instrumentation "i" therethrough (See FIG. 3). Longitudinal passage 108 is substantially parallel to longitudinal axis "a." The diameter and the shape of longitudinal passage 108 may vary. In one embodiment, the cross-section of longitudinal passage 108 has an octagonal shape. Irrespective of its shape or size, longitudinal passage 108 extends from a proximal end 110 of housing 102 to a distal end 112 of cannula sleeve 104.

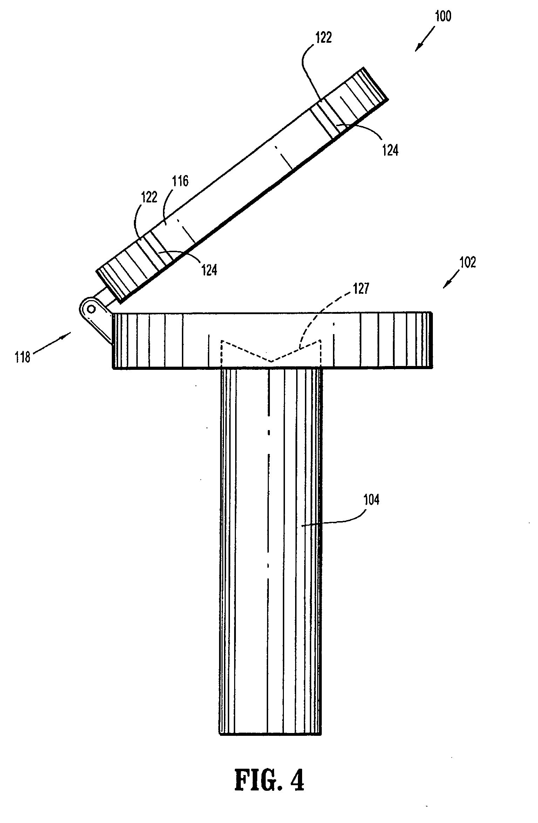

Suture management system 114 is operatively connected to housing 102. In general, suture management system 114 includes a suture retainer member 116 and a hinge 118. Hinge 118, or any other suitable mechanism, pivotally connects retainer member 116 to proximal end 110 of housing 102 and allows retainer member 116 to move between a closed position, as shown in FIG. 1, and an open position, as depicted in FIG. 4. Retainer member 116 defines an opening 120 disposed in fluid communication with longitudinal passage 108. Opening 120 is adapted to receive surgical instrumentation "i" (See FIG. 3). When a surgical instrument "i" is inserted through opening 120, retainer member 116 acts as a seal and forms a substantially fluid-tight seal around the surgical instrument "i" as seen in FIG. 3. Retainer member 116 may comprise an elastomeric material at least along locations adjacent opening 120 to flex and pennit passage of the surgical instrument in substantial sealed relation therewith. In certain embodiments of the present disclosure, housing 120 includes a second seal 127 shown in phantom in FIG. 4, which seals housing 102 in the absence of a surgical instrument. The second seal may be a zero closure valve, duckbill valve or the like. In addition to opening 120, retainer member 116 includes recesses 126 positioned along its outer perimeter. Recesses 126 are preferably arranged in spaced relation about the periphery of retainer member 116. In some embodiments, four recesses 126 are provided although more or fewer than four recesses 126 is envisioned.

Suture management system 114 contains at least one insert 122 disposed in each recess 126. In the depicted embodiment, suture management system 114 incorporates a plurality of inserts 122 positioned along the perimeter of flapper 116. Inserts 122 may be made of rubber or any other resilient material. Each insert 122 has a slit 124 extending from the outer perimeter thereof toward longitudinal axis "a" and extending longitudinally through the thickness of flapper 116. Slits 124 are each configured to receive and hold at least one suture "s." Further, slit 124 provides sutures "s" passage between the outside atmosphere and longitudinal passage 108 of access port 100. Those skilled in the art will recognize that inserts 122 may be attached to flapper 116 by any suitable means including via glue.

During operation, the suture management system 114 separates sutures "s" and reduces the risk of suture entanglement or confusion. Retainer member 116 of suture management system 114 serves as a seal and provides a substantially fluid-tight seal on the proximal end 110 of housing 102 when surgical instrumentation is inserted through opening 120. Overall, access port 100 provides passage between a subject's body cavity and the outside atmosphere.

In use, cannula sleeve 104 is positioned to access the targeted body site, e.g., the abdominal cavity in a laparoscopic procedure or an arthroscopic procedure. Surgical instrumentation is introduced through cannula sleeve 104 and into the surgical site through longitudinal passage 108. While the clinician inserts the surgical instrumentation "i" through longitudinal passage 108, retainer member 116 forms a substantially fluid-tight seal around the surgical instrumentation "i", thereby minimizing the exchange of gas or fluid such as saline in an arthroscopic procedure between the subject's body cavity and the outside atmosphere. Sutures "s" may be inserted into the subject's body cavity before, during, or after insertion of the surgical instrument "i". In certain surgical procedures, the sutures are attached to the surgical instrumentation "i". In any event, the clinician inserts the proximal regions of each suture "s" into slit 124 of a particular insert 122. At this moment, inserts 122 hold sutures "s" in place and consequently separate sutures "s" from each other, thereby reducing the risk of sutures "s" tangling with one another. The sutures are used to secure or tie down a tendon, ligament in soft tissue or the like. The clinician may pivot retainer member 116 with respect to housing 102 (see FIG. 4) to facilitate the insertion of the proximal regions of sutures "s" into slits 124 of inserts 122. To release sutures "s" from inserts 122, the clinician removes the proximal regions of sutures "s" out of each slit 124.

It will be understood that various modifications may be made to the embodiments of the presently disclosed access port. Therefore, the above description should not be construed as limiting, but merely as exemplifications of embodiments. Those skilled in the art will envision other modifications within the scope and spirit of the present disclosure.

## Claims

1. An access port for providing passage into a subject's body, which comprises:
a housing defining a longitudinal axis and having a longitudinal passage, the longitudinal passage being adapted to allow passage of a plurality of sutures through the housing; and
a suture management system operatively coupled to the housing, the suture management system including a flapper, the flapper having a plurality of spaced apart recesses, each recess including an insert disposed in mechanical cooperation therewith and each insert having a slit configured to receive and hold at least one suture of plurality of sutures.

2. The access port of claim 1, wherein the flapper is pivotally connected to the housing.

3. The access port of claim 1, wherein the flapper defines an opening in fluid communication with the longitudinal passage of the housing, the opening being adapted to receive a surgical instrument.

4. The access port of claim 3, wherein the flapper is configured to form a substantially fluid-tight seal around the surgical instrument when the surgical instrument is inserted through the opening.

5. The access port of any preceding claim, wherein the inserts each comprise a resilient material.

6. The access port of any of claims 1 to 4, wherein in inserts each comprise an elastomeric material.

7. The access port of any preceding claim, further comprising a cannula sleeve connected to the housing.

8. The access port of any preceding claim, wherein the recesses are positioned along a perimeter of the housing.

9. The access port of any preceding claim, further comprising a hinge pivotally connecting the flapper to the housing.

10. The access port of any preceding claim, wherein the slits extends from an outer perimeter of the insert toward the longitudinal axis defined by the housing.

11. A method for managing sutures, comprising the steps of:
providing an access port including a flapper positioned at a proximal end thereof, the flapper having a plurality of spaced apart recesses, each recess including an insert disposed in mechanical cooperation therewith, each inserts having a slit configured to receive and hold at least one suture of a plurality of sutures;
positioning at least a portion of the access port inside a subject's body; inserting the plurality of sutures inside the subject's body through the access port; and
placing at least a portion of each suture into a slit to hold and separate the sutures from one another.

12. The method of claim 11, wherein the step of placing at least a portion of each suture into a slit includes placing proximal regions of each suture into the slit.

13. The method of claim 11 or claim 12, further comprising the step of pivoting the flapper with respect to the housing.

14. The method of any of claims 11 to 13, further comprising the step of releasing the sutures from the inserts by removing each suture out of its respective slits.
